# EUROPEAN PATENT APPLICATION

(11) **EP 0 679 379 A1**
(43) Date of publication of application: **02.11.1995**
(21) Application number: 95105758.7
(22) Date of filing: 18.04.1995
(51) Int. Cl.: A61F 13/20

(54) **Tampon and method of forming same**

(30) Priority: 15.04.1994 US 229414
(71) Applicant: KIMBERLY-CLARK CORPORATION, Neenah, Wisconsin 54956 (US)
(72) Inventor: Rentmeester, Tammy Jo, Oshkosh, WI 54901 (US)
(74) Representative: Diehl, Hermann, Dr. Dipl.-Phys.

(57) **Abstract**

A catamenial tampon (40) having a protective finger sheath (42) is disclosed along with a method of forming the tampon (40). The tampon (40) includes an absorbent pledget (26) having an insertion end (30) and a trailing end (32). A cover is secured to the absorbent and at least partially encloses the pledget (26) such that it cannot be separated from it. The cover extends beyond the trailing end (32) of the pledget (26) in the form of a flexible sheath (42). The sheath is sized and configured to easily receive and encircle a finger (48) of the user and is designed to prevent soiling of the finger during insertion of the tampon into her vagina. The sheath (42) can collapse upon itself once the pledget (26) is inserted into the woman's vagina. The sheath (42) also provides a reliable means for removing the tampon from her vagina in a clean and easy manner. The method of forming the tampon is also disclosed. The method includes forming an absorbent ribbon having a predetermined width. Positioning the absorbent ribbon over a cover with a portion of the cover having a width greater than the width of the absorbent ribbon. The absorbent ribbon and the cover are then cut to a desired length and both materials are rolled into a cylindrically shaped softwind. The softwind has a portion of the cover material extending longitudinally outward from one end in the form of a flexible sheath (42). The softwind is then radially compressed into a pledget (26) and the insertion end (30) of the pledget (26) is rounded to facilitate it's insertion into a woman's vagina.

## Description

This invention relates to a catamenial tampon having a protective finger sheath and a method of forming such a tampon.

Currently, there are two basic types of catamenial tampons used for feminine hygiene. The first type is a digital tampon which is designed to be inserted into a woman's vagina directly by the user's fingers. The second type is a tampon which is designed to be inserted with the aid of an applicator. Both types are usually made by folding or rolling a loosely associated strip of absorbent into a cylindrical shape referred to as a "softwind" and then radially and/or biaxially compressing the softwind into a pledget. The pledget may or may not include a cover. In both types of tampons, a withdrawal string is attached to the softwind either before or after compression. The withdrawal string facilitates removal of the tampon from the user's vagina after it has absorbed menstrual fluid, blood, urine, etc.

It has been found that many women shy away from the digital style tampon because they can experience soiling of their fingers with body fluid while inserting a fresh tampon into their vagina. It has also been recognized that many women experience some difficulty in trying to locate and grasp the withdrawal string when they are ready to remove the tampon from their vagina. It is common for the withdrawal string to curl up and adhere to the distal end of the tampon. When the user searches for the string with her fingers, she finds that it is hard to locate. Once the withdrawal string is found, the user finds that her fingers have been soiled and may not have access to a sink where she can wash up.

Another problem is that sometimes the withdrawal string is separated from the pledget during the removal process. The user is then forced to pinch the tampon between her fingers and physically remove it or seek medical assistance. In the former situation, it is difficult for her to avoid soiling her fingers on the menstrual filled tampon.

Now a new tampon has been invented to overcome these frustrating inconveniences.

The general object of this invention is to provide a catamenial tampon having a protective finger sheath and a method of forming the tampon.

This object is solved by the tampon according to independent claim 1 and the methods according to independent claims 20 and 23. Further advantageous features, aspects, and details of the invention are evident from the dependent claims, and the description and the drawings. The claims are intended to be understood as a first non-limiting approach of defining the invention in general terms.

More particularly, this invention relates to a catamenial tampon designed to be worn by a female during her menstrual period to absorb menstrual fluid, blood, urine, etc.

Briefly, this invention relates to a catamenial tampon having a protective finger sheath and a method of forming the tampon. The tampon includes an absorbent pledget having an insertion end and a trailing end. A cover is secured to the absorbent and at least partially encloses the pledget such that it cannot be separated from it. The cover extends beyond the trailing end of the pledget in the form of a flexible sheath. The flexible sheath is sized and configured to easily receive and enclose a finger of the user and is designed to prevent soiling of the finger during the insertion of the tampon into the user's vagina. The flexible sheath also provides a reliable and clean means for removing the tampon from her vagina in an easy manner.

The method of forming the tampon with the protective finger sheath may include the following steps. First, an absorbent ribbon is formed having a predetermined width. Second, the absorbent ribbon is positioned or overlaid on a cover with a portion of the cover having a width greater than the width of the absorbent ribbon. Third, the absorbent ribbon and the cover are cut to a desired length and both materials are rolled into a cylindrically shaped softwind. The softwind has a portion of the cover extending longitudinally outward from one end in the form of a flexible sheath. Fourth, the softwind is radially compressed into a pledget and the insertion end of the pledget is rounded to facilitate insertion into a woman's vagina. The flexible sheath is in the form of a skirt and can be conical or tubular in configuration so as to receive and enclose a finger of the user. The flexible sheath spans a circular arc of at least 360 degrees and has sufficient length to prevent the finger from being soiled by body fluid when the tampon is inserted into the woman's vagina.

A specific aspect of this invention is to provide a catamenial tampon having a cover integrally attached to the absorbent.

Another aspect of this invention is to provide a catamenial tampon which does not require a withdrawal string.

A further aspect of this invention is to provide a catamenial tampon having a visually distinctive appearance.

Still another aspect of this invention is to provide a catamenial tampon which prevents the user's fingers from becoming soiled by body fluid either during insertion or removal of the tampon into and out of her vagina.

Still further, an aspect of this invention is to provide a tampon with a flexible finger sheath which can collapse once the tampon is inserted into the vagina cavity so as to be unnoticeable to the wearer.

Further features and advantages of this invention will become more readily apparent from the following detailed description when taken in conjunction with the accompanying drawings, in which:
Fig. 1 is a perspective view of an absorbent having a rectangular configuration.
Fig. 2 is a top view of a cover having an approximately L-shaped configuration.
Fig. 3 is a top view of the absorbent overlaying the approximately L-shaped cover.
Fig. 4 is a side view of a cylindrical softwind rolled up in direction "A" as indicated in Fig. 3 whereby the flexible sheath acquires a conical appearance.
Fig. 5 is a side view of a cylindrical softwind rolled up in direction "B" as indicated in Fig. 3 whereby the flexible sheath acquires a tubular appearance.
Fig. 6 is a schematic view depicting a woman's index finger positioned in the protective flexible sheath and ready to place the tampon into her vagina.
Fig. 7 is a side view of a tampon having a protective finger sheath along with a withdrawal string.
Fig. 8 is a perspective view of a tampon shown in Fig. 7 positioned in a tampon applicator.
Fig. 9 is a side view of an alternative embodiment wherein the flexible sheath has a conical appearance and is angularly offset from the pledget.
Fig. 10 is a top view of a cover having a modified L-shaped configuration to form a conical sheath that is offset at an angle from the pledget.
Fig. 11 is a flow diagram depicting a method of forming the tampon with a protective finger sheath.

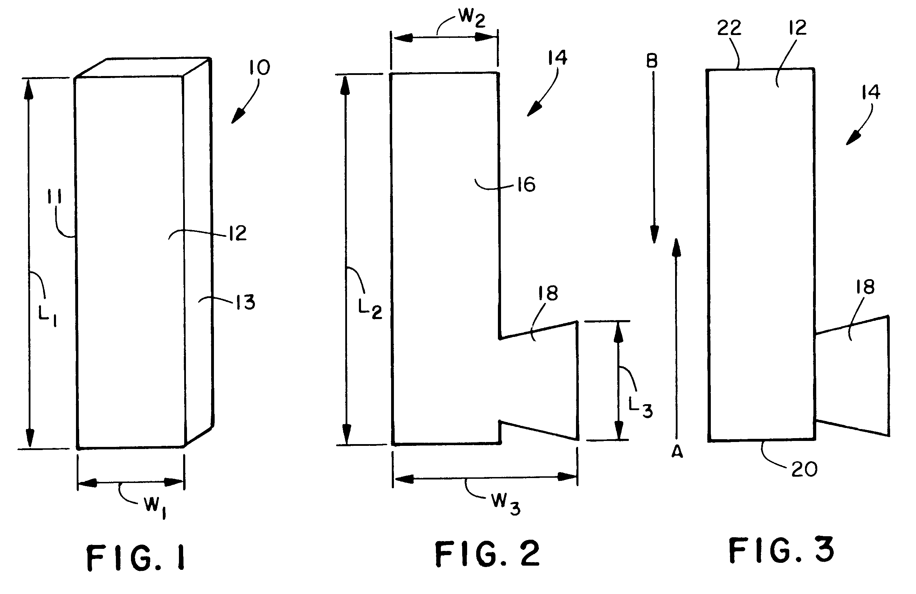

Referring to Fig. 1, an absorbent 10 is shown in the form of an elongated, rectangular ribbon 12 having a predetermined length L₁ and width W₁. The absorbent ribbon 12 has first and second longitudinal edges 11 and 13 which are approximately parallel to one another. The absorbent 10 can be made from natural or artificial fibers including polyester, cellulose, acetate, nylon, polypropylene, rayon, cotton or blends thereof. The absorbent 10 can also be a nonwoven, such as a bonded carded web, an airlayed web or a needle punched web. Such webs can be constructed of cotton and rayon fibers. A homogeneous blend of bleached cotton fibers and rayon fibers works well. The absorbent fibers can be formed by convolutely winding multiple fibers into a ribbon. The absorbent ribbon 12 can have a length L₁ ranging between about 102 mm to about 356 mm (about 4 to about 14 inches), preferably, about 152 mm to about 203 mm (about 6 to about 8 inches), most preferably, about 178 mm (about 7 inches). The width W₁ of the absorbent ribbon 12 can range between about 25 mm to 127 mm (about 1 to 5 inches), preferably about 51 mm to about 102 mm (about 2 to about 4 inches), most preferably, about 70 mm (about 2.75 inches). The thickness of the absorbent ribbon 12 can vary depending upon the diameter of tampon one wishes to manufacture. A thickness of between about 2 mm to 7 mm works well for a digital tampon.

Referring to Fig. 2, a liquid-permeable cover 14 is shown having an approximately L-shaped configuration with an upstanding leg 16 and a horizontal foot 18. The cover 14 can be constructed of a perforated or nonperforated nonwoven or a thermoplastic film which has been perforated to make it liquid-permeable. The cover 14 can have a thickness of between about 0.01 mm to about 1.0 mm, preferably less than about 0.5 mm, and most preferably, less than 0.3 mm. The thickness of the cover should be less than 50 percent of the thickness of the absorbent ribbon 12. Preferably, the thickness of the cover 14 is less than 10 percent of the thickness of the absorbent ribbon 12 and more preferably, less than 5 percent of the thickness of the absorbent ribbon 12.

The cover 14 can be treated with an emollient, a lubricant, or a surfactant to give it certain qualities. An emollient can be used to make the cover 14 softer and less abrasive. A lubricant can be used to facilitate insertion of the tampon into a woman's vagina. A surfactant can be used to allow body fluid to penetrate the cover 14 or to make the cover 14 more hydrophilic, that is, to increase it's affinity for absorbing fluids. It is also possible to treat a portion of the cover 14 to make it hydrophobic so that it will shed fluid. For example, the foot portion 18 of the cover 14 could be treated to be hydrophobic while the leg portion 16 of the cover 14 could be treated to be hydrophilic.

The cover 14 can be constructed of natural or synthetic materials and should be easily penetrated by body fluid, such as menstrual fluid, blood, urine, etc. Suitable materials include nonwovens, bonded carded webs of polyester, polypropylene, polyethylene, nylon or other heat-bondable fibers. Other polyolefins, such as copolymers of polypropylene and polyethylene, linear low density polyethylene, finely perforated thermoplastic films and net materials, also work well. A preferred material is spunbond which is manufactured and sold by Kimberly-Clark Corporation. Another material that also works well is a neck-bonded laminate manufactured by Kimberly-Clark Corporation. The neck-bonded laminate is constructed of an elastomeric film, like Kraton®, which is thermally laminated in an unstretched state between two spunbond facing sheets. The neck-bonded laminate has sufficient strength as well as the ability to stretch in the cross direction instead of in the machine direction. Having a cover material that can stretch in only one direction is advantageous when forming the flexible sheath.

The cover 14 has an overall length L₂ which can be less than, equal to, or greater than the length L₁ of the absorbent ribbon 12. The cover 14 also has a width W₂ corresponding to the width of the leg 16 and a width W₃ corresponding to the width of the foot 18. In this approximately L-shaped configuration, the width W₂ can be less than, equal to, or greater than the width W₁ of the absorbent ribbon 12 while the width W₃ of the foot should be greater than the width W₁ of the absorbent ribbon 12. Preferably, the width W₂ of the leg 16 will be less than or equal to the width W₁ of the absorbent ribbon 12 and the width W₃ of the foot will be greater than the width W₁ of the absorbent ribbon 12. It should be noted that the cover 14 could be cut to a general T-shape or to some other configuration if desired. The invention is being described with the cover 14 having an approximately L-shaped configuration for illustration purposes only.

The cover 14 could also be cut in the form of a rectangle, a square, or some other geometrical configuration. If the cover 14 were in the shape of a rectangle, then it could have a single width with at least a portion of the width being less than, equal to or greater than the width W₁ of the absorbent ribbon 12. For example, the width W₃ of the cover 14 could be equal to or less than the width W₁ of the absorbent ribbon 12 but the cover 14 could be transversely offset with respect to the absorbent ribbon 12 such that one longitudinal side edge of the cover 14 extends beyond one of the longitudinal side edges of the absorbent ribbon 12.

Referring again to Fig. 2, the foot portion 18 of the cover 14 is depicted as having a trapezoid profile. Although this design forms a preferred sheath, a square, a rectangle or other type of configuration is acceptable. The length L₃ of the foot portion 18 can extend a distance which is between about 20 to 100 percent of the overall length L₂ of the cover 14. Preferably, L₃ will range from between about 20 to about 50 percent of the length L₂ of the cover 14. It has been found that by making L₃ only a fraction of the total length L₂, less cover material 14 has to be used and this could reduce the overall cost required to produce the tampon. A second benefit is that the finished tubular or conical shaped sheath will not contain multiple loops of cover material and thus it will be easier for a woman to insert her finger into the flexible sheath.

Referring to Fig. 3, the absorbent ribbon 12 is positioned over and aligned with the cover 14. The absorbent ribbon 12 and the cover 14 are depicted as having approximately the same length. However, the absorbent ribbon 12 can have a length which is equal to or different from the length of the cover 14. Furthermore, both the absorbent ribbon 12 and the cover 14 are shown aligned such that they each have a first end 20 and a second end 22 which coincide with one another. However, it is possible to longitudinally and/or transversely offset the absorbent ribbon 12 from the cover 14, if desired. The particular alignment will depend upon the final pledget design one desires. For example, some manufacturers prefer to expose the insertion end of the absorbent 12 to facilitate absorption of body fluid. In this case, it is not necessary to align the cover 14 relative to the absorbent 12 such that the insertion end would be enclosed.

It is possible to align the absorbent ribbon 12 on the cover 14 such that at least a portion of the cover 14 extends over at least one of the longitudinal side edges 11 or 13 of the absorbent ribbon 12. This is an alternative to having to construct the cover 14 with a width at least a portion of which is greater than the width of the absorbent ribbon 12.

Once the absorbent ribbon 12 and the cover 14 are mated together, they can be radially wound, rolled up or folded into a cylindrical shape known as a "softwind" by starting from either the first end 20 or the second end 22. A "softwind" is a term known to those skilled in the art of designing and manufacturing tampons which means a radial wound absorbent ribbon, with or without a cover, before it is radially compressed into a pledget. A "pledget" is a radially compressed softwind.

The diameter of the softwind can vary depending upon the starting thickness of the absorbent ribbon 12, the thickness of the cover 14, the degree to which the absorbent ribbon 12 has been radially wound, the desired diameter of the compressed pledget, etc. US-A-4,951,368 issued August 28, 1990 to Heinen, and assigned to the present assignee, teaches an apparatus for compressing an absorbent into a tampon. This patent is incorporated by reference and made a part hereof.

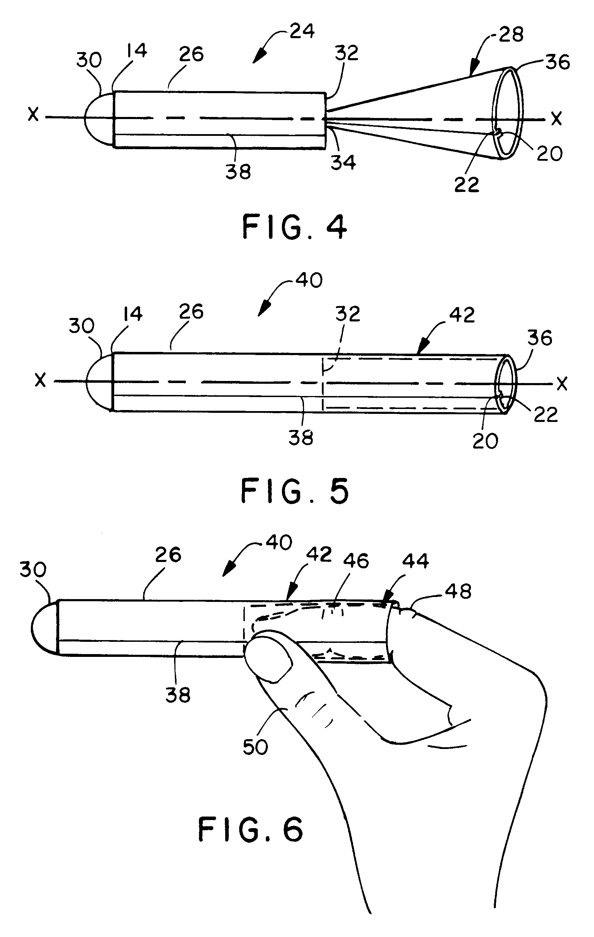

Referring to Fig. 4, a catamenial tampon 24 is shown which is formed by forming a softwind by radially winding or rolling up the absorbent ribbon 12 and cover 14 in the direction indicated as "A" in Fig. 3, and then compressing the softwind into a pledget 26. The tampon 24 includes a flexible sheath 28 having a conical configuration which is integrally secured to the absorbent pledget 26. The absorbent ribbon 12 and the cover 14 will be bonded together by heat, pressure or a combination of heat and pressure during the compression step and therefore cannot be separated without destroying the tampon 24. The pledget 26 has an insertion end 30 spaced apart from a trailing end 32. The insertion end 30 is not covered by the cover 14 so as to allow for a faster absorption of body fluid once the tampon 24 is placed inside a woman's vagina. The insertion end 30 can be rounded or pointed to form an angled nose, for example, a semi-spherical shaped nose, which will facilitate insertion of the tampon 24 into a woman's vagina. Covering and extending longitudinally outward from the trailing end 32 of the pledget 24 is the flexible sheath 28. The flexible sheath 28 is an extension of the cover 14 and forms a hollow skirt which acquires a conical shape when opened to receive a user's finger. The portion of the cover 14 surrounding the pledget 26 is integral with the portion of the cover 14 which forms the sheath 28. The cover 14 encloses the second end 32 of the pledget 26 and is gathered adjacent to the longitudinal axis X--X of the tampon 24. This point is located approximately at an apex 34 of the conical sheath 28. The sheath 28 has a flexible wall 36 which angles radially outward as it moves away from the trailing end 32 of the pledget 26. This flexible wall 36 forms a cone having an internal diameter which is large enough to receive and enclose a portion of a user's finger.

The flexible sheath 28 is radially wound in direction "A", as depicted in Fig. 3, such that the first end 20 becomes the inside edge and the second end 22 becomes the outside edge. The two ends 20 and 22 form a circular arc which spans at least 360 degrees such that the second end 22 at least abuts the first end 20. Preferably, the second end 22 will overlap the first end 20. The overlap can be obtained by having the wall 36 span an arc slightly greater than 360 degrees or by having the wall 36 span an arc up to about 1,080 degrees. Three concentric windings of the wall 36 will provide an arc of 1,080 degrees. The exact amount of overlap will depend upon the length of the cover 14 and how it is aligned relative to the absorbent ribbon 12. It is possible to have more than three complete circular arcs if desired. However, the presence of multiple 360 degree windings could interfere with the insertion of a finger into the sheath 28. When the cover 14 is made of a very thin material, multiple wrappings decrease the ease of insertion of a finger into the sheath 28 and this is to be avoided.

The overlap of the ends 20 and 22 of the wall 36 assures that the circumference of the user's finger, which will be positioned in the sheath 28, will be completely protected from being soiled by body fluid. If it wasn't for the flexible sheath 28, a woman could encounter menstrual fluid, blood, urine, etc. during the insertion process which could soil her fingers as she inserts the tampon.

The conical sheath 28 should have an outside diameter at it's free end which is larger than the outside diameter of the pledget 26. This size difference will facilitate insertion of the woman's finger into the sheath 28. It should also be mentioned that the sheath 28 is very flexible and the wall 36 will collapse upon itself when the tampon 24 is inserted into the woman's vagina. The collapse of the sheath 28 is beneficial in that it cannot be detected by the wearer and therefore provides for a more comfortable product. The thinnest of the cover 14 contributes to the flexibility of the sheath 28 and allows it to collapse once the woman's finger is withdrawn.

It should be mentioned that it is possible to treat only a certain portion of the cover 14 or to perforate only a given area of the cover 14, if desired. For example, an emollient or a surfactant could be applied to only that portion of the cover 14 which surrounds the pledget 26. Furthermore, it is not necessary to perforate the portion of the cover 14 which forms the sheath 28 because one does not want body fluid to penetrate through the sheath 28. It is also not desirable to lubricate the portion of the cover 14 which forms the sheath 28 because one does not want the user's finger to slip out of the sheath 28 during the insertion process. Likewise, it is not necessary to treat the sheath 28 with a hydrophilic surfactant. However, the sheath 28 could be treated to prevent or inhibit fluid penetration with a material such as wax.

Referring again to Fig. 4, the cover 14 can be secured to the absorbent ribbon 12 over the entire length of the pledget 26 during the compression step by a seam 38. In addition, the cover 14 can be secured to itself over at least a portion of the length of the flexible sheath 28 by an extension of the seam 38. The seam 38 will ensure that the second or outer end 22 of the cover 14 does not move away from abutting or overlapping the first or inner end 20 of the cover 14. The seam 38 can be formed by applying heat, pressure, or the combination of heat and pressure. Furthermore, the seam 38 can be formed by using an adhesive, by sewing, by ultrasonic bonding, or by any other means known to those skilled in the art. The seam 38 can be intermittent or continuous, depending on the particular manufacturing process employed, and does not have to extend over the total width W₃ of the cover 14.

It should also be noted that along the length of the flexible sheath 28, the seam 38 can be formed by a different process than that used to form the seam 38 along the length of the pledget 26. For example, the seam 38 can be formed over the length of the pledget 26 by using heat and pressure to bond the cover 14 to the absorbent ribbon 12 while an ultrasonic bond can be used to form the seam 38 over the length of the sheath 28.

Referring to Fig. 5, a catamenial tampon 40 is shown which is formed by forming a "softwind" by folding, radially winding or rolling up the absorbent ribbon 12 and cover 14 in the direction indicated as "B" in Fig. 3, and then compressing the softwind into a pledget 26. The tampon 40 includes a flexible sheath 42 integrally secured to the absorbent pledget 26. The pledget 26 is identical to that described above. The pledget 26 has an insertion end 30 spaced apart from a trailing end 32. The difference in Fig. 5 is that the cover 14 extends longitudinally outward from the outer circumference of the pledget 26 to form a hollow, tubular sheath 42. The outside diameter of the tubular sheath 42 is essentially constant and equal to the outside diameter of the pledget 26. The inside diameter of the tubular sheath 42 should be of a sufficient size to allow the insertion of a woman's finger or thumb into it. The tubular configuration of the sheath 42 is an option to the conical configuration sheath 28 shown in Fig. 4. However, both sheaths 28 and 42 functions in a similar fashion.

The tubular sheath 42 has a flexible wall 36 which spans an arc of at least 360 degrees and the ends of the wall 36 can be secured together by a seam 38. The seam 38 can extend along the length of the pledget 26 as well as along the length of the sheath 42 to ensure that the first or outer end 20 does not move away from abutting or overlapping the second or inner end 22 of the cover 14. The seam 38 can be formed as stated above.

Referring to Fig. 6, a schematic view is shown depicting a woman's index finger 44 positioned inside the tubular sheath 42 of the tampon 40. The flexible sheath 42 has a length equal to W₃ minus W₂, shown in Fig. 2. This length should be long enough to enclose one of the user's fingers or thumb, preferably the index or middle finger, and extend pass the fingernail and up to the first knuckle 46. Preferably, the flexible sheath 42 has a sufficient length to approach and possible cover the second knuckle 48. The flexible sheath 42 should extend longitudinally outward from the cylindrically shaped pledget 26 for a distance equal to at least half the width of the cylindrically shaped pledget 26. For example, the flexible sheath 42 can have a length of at least 12.7 mm (0.5 inches), preferably, at least 25.4 mm (1.0 inches), and most preferably, at least 38.1 mm (1.5 inches). This distance will vary for each woman and therefore the preferred length will depend on the preference of each manufacturer. Another way of constructing the tampon 40 is to make the length of the sheath 42 approximately equal to the length of the pledget 26.

It should be realized that even though the index or middle fingers are the ones most likely to be used by a woman to insert the tampon 24, some women may prefer to use a different finger or even the thumb. Any finger, including the thumb, should be accommodated by the inner opening of the flexible sheath 42. As explained above, after the tampon 40 is inserted into a woman's vagina, the sheath 42 will collapse upon itself and be virtually unnoticeable by the wearer.

It should be noted that the thumb 50 can be used to squeeze the flexible sheath 42 against the tip of the index finger 44 so as to control the orientation of the pledget 26 as it is initially positioned at the opening of the vagina. Some women may find it desirable to position the tip of the thumb at the base of the pledget 26 to gain greater control. Likewise, some women may find the insertion process to be easier if they place their middle finger in the flexible sheath 42 and use both their thumb and ring finger on the outside of the sheath 42. The thumb and ring finger would be withdrawn after the pledget 26 is aligned with the opening of the vagina and only the middle finger would be used to insert the pledget 26 into the vagina. It is also possible for a user to twist the flexible sheath 42 once her finger is inserted into it, so as to obtain greater control of the tampon 40. The flexible sheath 42 should be sized and configured to accommodate these various styles of insertion.

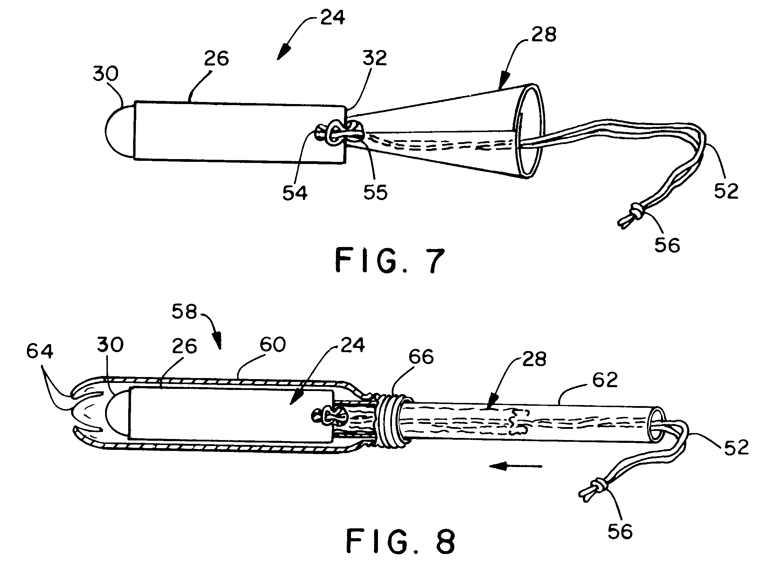

Referring to Fig. 7, an alternative embodiment of the tampon 24 is shown. In this embodiment, a withdrawal string 52 is securely attached to the pledget 26 via an opening 54 formed therein. The withdrawal string 52 passes through the opening 54 and is looped upon itself. The withdrawal string 52 then passes through a second opening 55 formed in the sheath 28 and extends through the interior of the sheath 28. The free ends of the withdrawal string 52 terminate outside of the flexible sheath 28 where they are secured together by a knot 56. The knot 56 insures that the withdrawal string 52 cannot be separated from the pledget 26. The withdrawal string 52 can contain a wax coating to prevent it from absorbing body fluid. The flexible sheath 28 shelters the withdrawal string 52 from contacting the inside walls of the woman's vagina and thereby serves to keep the withdrawal string 52 clean and dry so that it can be used to withdraw the tampon 24 without soiling the fingers of the user. The flexible sheath 28 also functions to provide a backup means for withdrawing the pledget 26 should the withdrawal string 52 break.

Referring to Fig. 8, the tampon 24 is shown positioned within a tampon applicator 58. One type of tampon applicator is taught in US-A-5,158,535 issued to Paul et al. on October 27, 1992 and is assigned to the present assignee. This patent is incorporated by reference and made a part hereof. The tampon applicator 58 is constructed of an outer tube 60 and an inner tube 62. The outer tube 60 is sized and configured to house the pledget 26 and includes a plurality of flexible petals 64 formed on the forward or expulsion end. The petals 64 are designed to flex or bend outward to provide an opening through which the pledget 26 can be ejected from the outer tube 60 at the appropriate time. The opposite end of the outer tube 60 narrows into a fingergrip portion 66 which provides a surface which can accommodate the user's thumb and middle finger so that the outer tube 60 can be properly positioned in the user's vagina. The inner tube 62 is a hollow member which is telescopically movable within the fingergrip portion 66 of the outer tube 60. When the inner tube 62 is pushed forward into the outer tube by the user's index finger, it will contact the pledget 26 and expel it through the opening formed when the petals 64 separate. As this occurs, the flexible sheath 28 and the withdrawal string 52 pass through the hollow inner and outer tubes, 62 and 60 respectively. The tampon applicator 58 can then be discarded.

It should also be mentioned that the tampon 24 can have an opening formed in it's second end 32 which is designed to receive a stick. The stick serves as an applicator and can be removed once the tampon 24 is positioned in the user's vagina. US-A-3,683,912 issued to Olson et al. and assigned to the present assignee teaches such a tampon. This patent is incorporated by reference and made a part hereof.

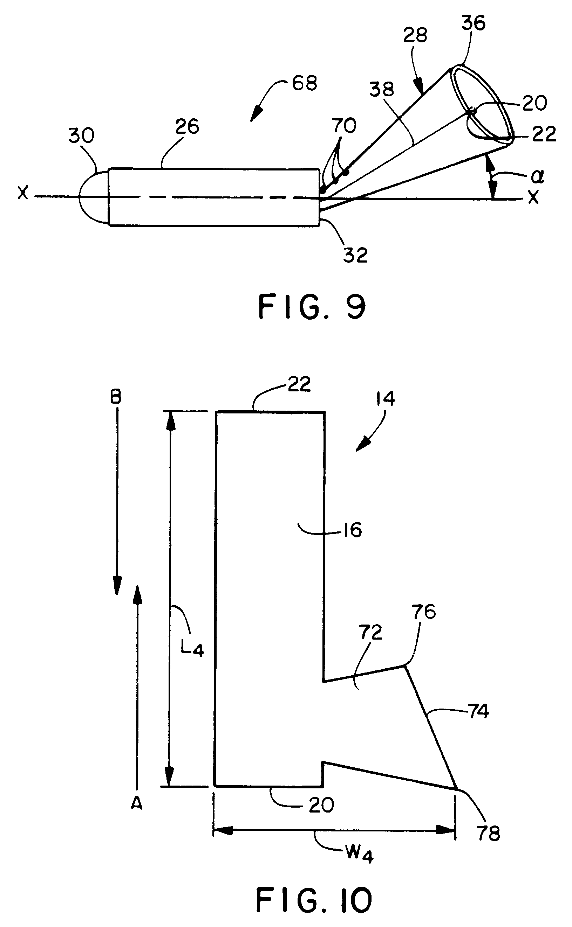

Referring to Fig. 9, an alternative embodiment of a tampon 68 is shown wherein the flexible sheath 28 is offset from the longitudinal axis X--X by an angle alpha (α). The angular offset can facilitate positioning the tampon 68 into a woman's vagina. This angular offset can be obtained a number different ways. One way is to apply a spot of adhesive 70, such as a construction glue, to bond the sheath 28 to the second end 32 of the pledget 26. The adhesive 70 can be applied as one or more dots, or as a continuous line or seam, to that the portion of the sheath 28 which is needed to hold it at a desired angle. The angle can vary from about 10 degrees to about 75 degrees with an angle of between about 20 degrees to 45 degrees working the best.

Referring to Fig. 10, a second way to obtain the sheath 28 at an angular orientation to the pledget 26 is to cut the cover 14 as shown. In Fig. 10, the cover 14 includes a rectangular shaped leg 16 having an odd shaped foot 72 extending outward therefrom. The leg 16 and foot 72 form an approximately L-shaped cover 14 with a length L₄ and a width W₄. The difference is that the foot 72 has an edge 74 formed by a line joining points 76 and 78. The edge is formed at an angle to the longitudinal side edge of the cover 14 so that when the cover 14 is rolled up, the sheath will fold or bend at the second end 32 and exhibit the angular offset. The offset can be enhanced by the addition of the seam 38 which secured the free ends 20 and 22 of the sheath together. In addition, it should be mentioned that a spot of adhesive too can also be utilized, as shown in Fig. 9, to maintain the desired angular offset.

### METHOD

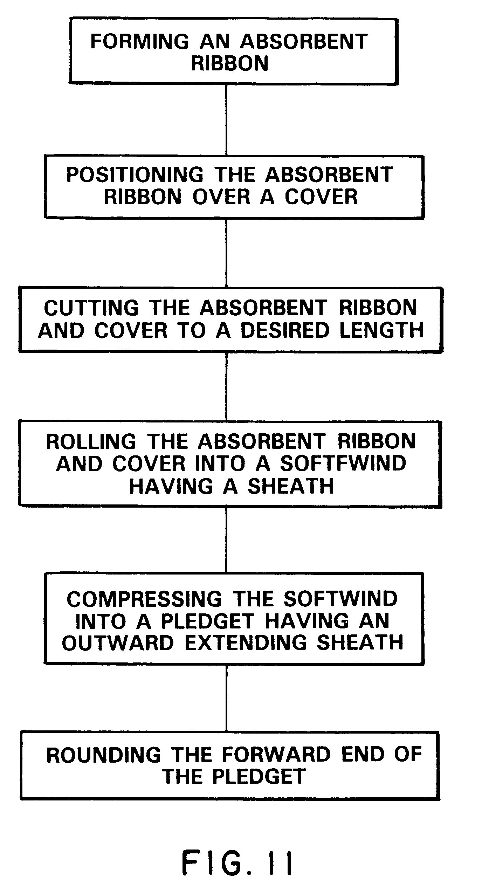

Referring to Fig. 11, a flow diagram is shown depicting a method of forming the catamenial tampon having a protective finger sheath 28. The method includes first, forming an absorbent ribbon 12 having first and second longitudinal edges, 11 and 13 respectively, and having a predetermined width W₁. Second, positioning the absorbent ribbon 12 on a cover 14 such that at least a portion of the cover 14 extends over at least one of the longitudinal edges, 11 or 13, respectively. Another way of accomplishing this is to construct the cover 14 such that it has a width greater than the width of the absorbent ribbon 12. Third, the absorbent ribbon 12 and the cover 14 are cut to a desired length. Fourth, the absorbent ribbon 12 and the cover 14 are folded, radially wound or rolled up into a cylindrical "softwind" wherein at least a portion of the cover 14 extends longitudinally outward from the softwind and forms an integral sheath 28. The flexible sheath 28 can have a tubular or conical configuration and is hollow on the inside. Preferably, the wall 26 of the sheath 28 spans an arc of at least 360 degrees and the arc is circular in configuration. Fifth, the softwind is radially compressed into a pledget 26 having an insertion end 30 and a trailing end 32. Lastly, the insertion end 30 is rounded to facilitate insertion of the tampon 24 into a woman's vagina.

It should be noted that the last step of the above described method can be eliminated if desired. It should also be noted that the sequence of some of the steps may be altered depending upon the equipment used to manufacture the tampon 24.

While the invention has been described in conjunction with several specific embodiments, it is to be understood that many alternatives, modifications and variations will be apparent to those skilled in the art in light of the aforegoing description. Accordingly, this invention is intended to embrace all such alternatives, modifications and variations which fall within the spirit and scope of the appended claims.

## Claims

1. A tampon comprising:
a) a pledget (26) having first (30) and second ends (32); and
b) a cover (14) at least partially enclosing said pledget and extending beyond said second end to form a sheath (28,42), said sheath (28,42) being capable of receiving a user's finger and preventing soiling of the finger during insertion of said tampon into a body cavity, said sheath capable of collapsing upon itself once said pledget is inserted into said body cavity.

2. The tampon according to claim 1 wherein said sheath further provides means for removing said tampon from said body cavity.

3. The tampon according to claim 1 or 2 wherein said pledget (26) is an absorbent pledget.

4. The tampon according to any of the preceding claims wherein said sheath (28,42) is a flexible sheath (28,42).

5. The tampon according to any of the preceding claims wherein said first and second ends are an insertion end (30) and a trailing end (32).

6. The tampon according to any of the preceding claims wherein said tampon is a catamenial one.

7. The tampon according to any of the preceding claims, wherein said cover (14) is integrally secured to said pledget (26).

8. The tampon according to any of the preceding claims, wherein said cover (14) is a nonwoven.

9. The tampon according to any of claims 1 to 7, wherein said cover is an apertured thermoplastic film.

10. The tampon according to any of the preceding claims wherein said cover (14) is liquid permeable.

11. The tampon according to any of the preceding claims wherein said cover (14) is treated with a surfactant.

12. The tampon according to any of the preceding claims wherein said sheath (28,42) extends longitudinally outward from said second end of said pledget (10).

13. The tampon according to any of the preceding claims wherein said sheath (28,42) extends longitudinally outward from said pledget (26) for a distance equal to at least half the length of said pledget (26).

14. The tampon according to any of the preceding claims wherein said sheath (28,42) extends longitudinally outward from said pledget (26) for a distance of at least 2.5 cm (1 inch).

15. The tampon according to any of the preceding claims wherein said sheath (42) has a tubular configuration.

16. The tampon according to any of claims 1 to 14 wherein said sheath (28) has a conical configuration.

17. The tampon according to any of the preceding claims wherein said sheath (28,42) spans an arc of at least 360°.

18. The tampon according to any of the preceding claims, wherein said pledget (26) is formed from an absorbent ribbon (12) and said cover (14) has a thickness which is less than about 5 percent of the thickness of said absorbent ribbon (12).

19. The tampon according to any of the preceding claims wherein a withdrawal string (52) is secured to said pledget (26) and extends outward through said sheath (28,42).

20. A method of forming a catamenial tampon comprising:
a) forming an absorbent ribbon (12) having first and second longitudinal edges;
b) positioning said absorbent ribbon (12) on a cover (14), at least a portion of said cover (14) extending over at least one of said longitudinal edges;
c) cutting said absorbent ribbon (12) and said cover (14) to a desired length;
d) rolling said absorbent ribbon (12) and said cover (14) into a cylindrical softwind wherein at least a portion of said cover (14) extends longitudinally outward from said softwind and forms a flexible sheath (28,42); and
e) compressing said softwind into a pledget having an integrally secured protective finger sheath (28,42).

21. The method of claim 20 wherein said absorbent ribbon (12) has a first end and a second end and said cover (14) has an approximately L-shaped configuration with a leg (16) and a foot (18), said foot (18) having a length which extends a distance at least about 20 percent along the length of said absorbent ribbon (12), said absorbent ribbon (12) positioned over said cover (14) such that said first end of said absorbent ribbon (12) coincides with said foot (18) of said L-shaped cover (14) and said absorbent ribbon (12) and said cover (14) are rolled up starting from said first end.

22. The method of claim 20 or 21 wherein said absorbent ribbon (12) has a first end and a second end and said cover (14) has an approximately L-shaped configuration with a leg (16) and a foot (18), said foot (18) having a length which extends a distance at least about 20 percent along the length of said absorbent ribbon (12), said absorbent ribbon (12) positioned over said cover (14) such that said first end of said absorbent ribbon (12) coincides with said foot (18) of said L-shaped cover (14) and said absorbent ribbon (12) and said cover (14) are rolled up starting from said second end.

23. A method of forming a catamenial tampon comprising:
a) forming an absorbent ribbon (12) having a predetermined width;
b) positioning said absorbent ribbon (12) on a cover (14), at least a portion of said cover (14) having a width greater than the width of said absorbent ribbon (12);
c) cutting said absorbent ribbon (12) and said cover (14) to a desired length;
d) rolling said absorbent ribbon (12) and said cover (14) into a cylindrical softwind wherein at least a portion of said cover (14) extends longitudinally outward from said softwind and forms a flexible sheath (28,42), said sheath (28,42) spanning an arc of at least 360 degrees;
e) radially compressing said softwind into a pledget having an insertion end and a trailing end with said protective finger sheath (28,42) extending out from said trailing end; and
f) rounding said insertion end of said pledget to form a tampon which can be comfortably inserted into a body cavity.

24. The method of claim 23 wherein said absorbent ribbon (12) and said cover (14) are rolled such that said sheath (28) acquires a conical configuration having an apex positioned adjacent to an end of said pledget.
